# EUROPÄISCHE PATENTSCHRIFT

(11) **EP 1 160 830 B1**
(45) Veröffentlichungstag und Bekanntmachung des Hinweises auf die Patenterteilung: **10.05.2006**
(21) Anmeldenummer: 01113299.0
(22) Anmeldetag: 31.05.2001
(51) Int. Cl.: H01J 61/35, H01J 61/42, H01J 61/72

(54) **Niederdruckentladungslampe**
Low pressure discharge lamp
Lampe à décharge à basse pression

(30) Priorität: 31.05.2000 DE 20009687 U
(43) Veröffentlichungstag der Anmeldung: 05.12.2001
(73) Patentinhaber: SLI Lichtsysteme GmbH, 91056 Erlangen (DE)
(72) Erfinder: Müller, Bernd, Dipl.-Ing., 91315 Höchstadt (DE)
(74) Vertreter: Lemke, Jörg-Michael

(56) Entgegenhaltungen:
- EP-A- 0 228 737
- US-A- 3 275 872
- US-A- 3 821 578
- US-A- 4 317 066
- PATENT ABSTRACTS OF JAPAN vol. 008, no. 272 (E-284), 13. Dezember 1984 (1984-12-13) & JP 59 141159 A (SUWA SEIKOSHA KK), 13. August 1984 (1984-08-13)
- PATENT ABSTRACTS OF JAPAN vol. 012, no. 403 (E-674), 26. Oktober 1988 (1988-10-26) & JP 63 146340 A (TOSHIBA CORP), 18. Juni 1988 (1988-06-18)

## Beschreibung

Die Erfindung betrifft eine Niederdruckentladungslampe für Bräunungszwecke mit endseitigen Elektroden in einer rohrförmigen Hülle aus Glas oder dergleichen, auf deren Innenfläche eine Reflexionsschicht aufgebracht und anschließend von einer Leuchtstoffschicht überdeckt worden ist, wobei vor dem Überdecken durch die Leuchtstoffschicht dafür gesorgt wurde, daß zumindest ein Teil der Innenfläche in Längsrichtung des Rohrs schlitzartig von der Reflexionsschicht frei ist, derart, daß das im Betrieb aus dem vorhandenen Schlitz austretende sichtbare und/oder UV-Licht durch die Reflexionsschicht verstärkt ist.

Eine Lampe dieser Art für reprographische Zwecke ist aus der US-A 3 275 872 bekannt und besitzt einen Schlitz, der noch vor den Elektroden endet. Gemäß dieser Druckschrift ist ein solcher Schlitz jedoch nur dann nötig, wenn die Leuchtdichte über die gesamte Länge der Lampe möglichst gleichmäßig sein soll.

Eine Lampe für Bräunungszwecke ist aus der EP-A-0 228 737 bekannt. Aus dieser Druckschrift geht nicht hervor, daß sich das Problem der gleichmäßigen Leuchtdichte auch bei einer Bräunungslampe stellt.

Bei einer Bräunungslampe besitzt üblicherweise der vorhandene Schlitz in Umfangsrichtung des Glasrohrs oder dergleichen eine Breite, die in Ansehung des Rohrquerschnitts etwa 150 Grad beträgt. Mit anderen Worten: Die Reflexionsschicht überdeckt etwa 210 Grad in Umfangsrichtung der Innenfläche des Glasrohrs.

Die Elektroden einer solchen Niederdruckentladungslampe liegen zumeist als sogenannte Wendeln vor, gegebenenfalls auch Doppelwendeln. Im Bereich solcher Elektroden kann nun nicht nur ein sogenanntes Wendelflackern auftreten, das naturgemäß einen Betrachter stören kann, sondern es treten im Laufe des Betriebes nachgerade zwangsläufig im Elektrodenbereich Ablagerungen staubförmigen Wendelmaterials auf der benachbarten Innenfläche des Glasrohrs bzw. der Hülle auf. Derartige sogenannte Endschwärzungen, werden ebenfalls als störend empfunden.

Die der Erfindung zugrundeliegende Aufgabe wird nun darin gesehen, eine Niederdruckentladungslampe der eingangs genannten Bauart zu schaffen, bei welcher sowohl auftretendes Wendelflackern als auch die sogenannten Endschwärzungen nicht mehr stören.

Diese Aufgabe wird erfindungsgemäß dadurch gelöst, daß der Schlitz beidseitig vor den Elektroden endet, derart, daß ab jeweils Rohrende zumindest der Elektrodenbereich der rohrförmigen Hülle über 360° ihres Umfangs durch die Reflexionsschicht abgedeckt ist.

Unabhängig davon, ob beim Herstellungsverfahren zunächst die gesamte Innenfläche einer Hülle mit der Reflexionschicht versehen und anschließend der erforderliche Schlitz durch Entfernung der Reflexionsschicht in dem betreffenden Bereich geschaffen wird, oder ob der Bereich des Schlitzes von vorneherein bei der Beschichtung mit dem Material der Reflexionsschicht freigelassen wird, wird erfindungsgemäß stets dafür gesorgt, daß sich vom jeweiligen Hüllrohrende bis mindestens über den Elektrodenbereich eine geschlossene Reflexionsschicht erstreckt. Anders ausgedrückt ist in diesem Bereich die Reflexionsschicht über 360° der Innenfläche bzw. des inneren Umfangs der Hülle bzw. des Glasrohrs der Lampe angeordnet.

Als zusätzlicher Vorteil der erfindungsgemäßen Ausbildung wird angesehen, daß sie außerdem zu einer Regulierung des Wärmehaushaltes im Elektrodenbereich führt.

Die Erfindung wird im folgenden anhand einer in den Figuren der Zeichnung dargestellten, bevorzugten, Ausführungsform näher erläutert.

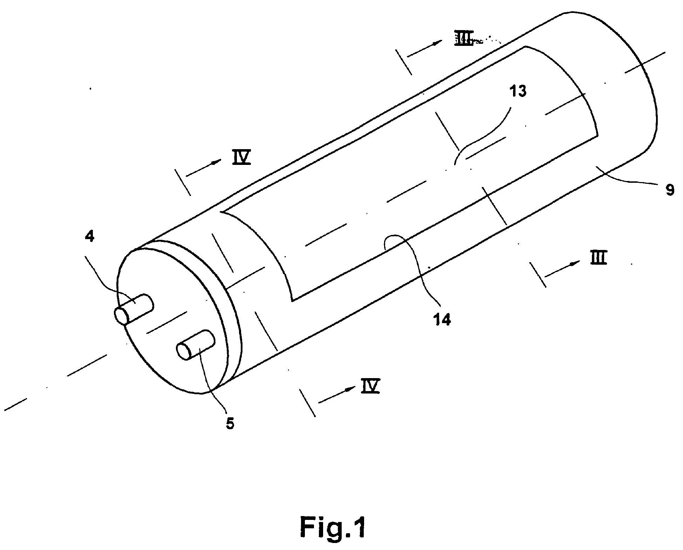

Es zeigt:
Fig. 1
   eine Ausführungsform einer Niederdruckentladungslampe nach der Erfindung in schaubildlicher Ansicht;
Fig. 2
   eine Draufsicht auf die Niederdruckentladungslampe nach Fig. 1 mit in Längsrichtung mittig aufgeschnittener Hülle mit Reflektorschicht und Leuchtstoffschicht;
Fig. 3
   einen Schnitt längs der Linie III-III in Fig. 1;
Fig. 4
   einen Schnitt längs der Linie IV-IV in Fig. 1.

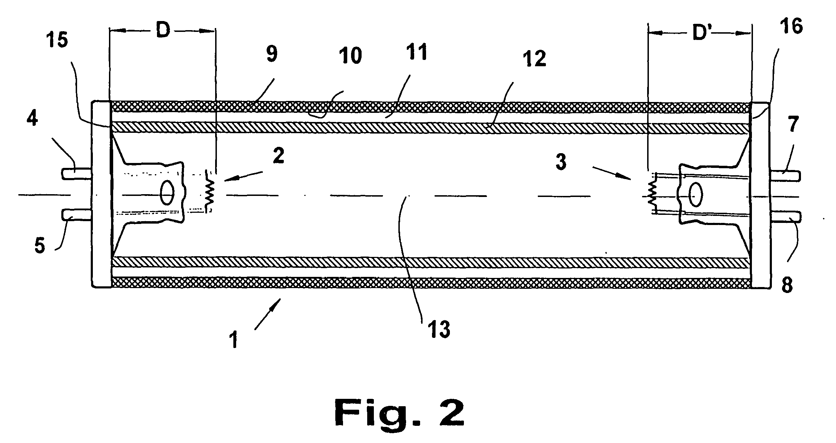

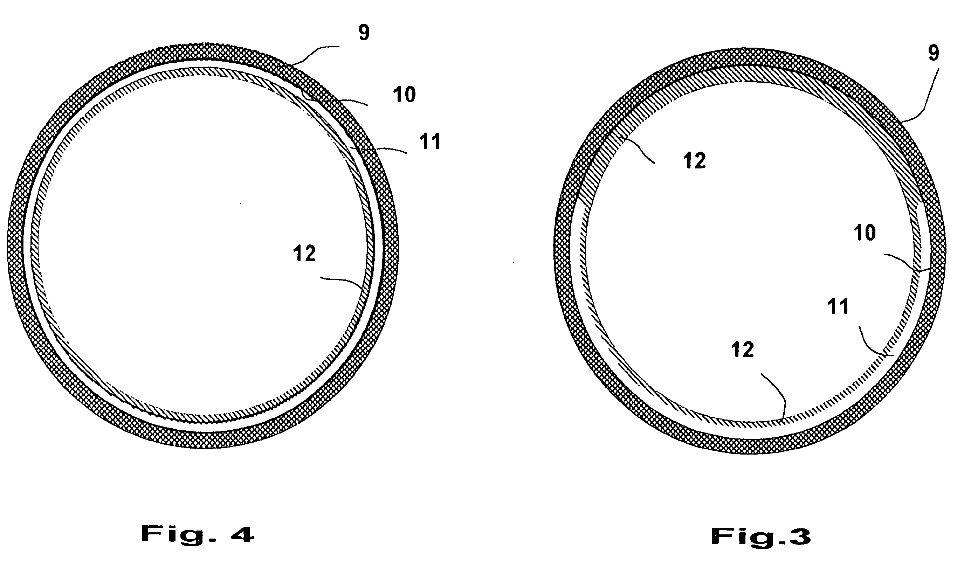

Fig. 1 zeigt eine Niederdruckentladungslampe 1, insbesondere eine UV-Bräunungslampe mit endseitigen Elektroden 2 und 3 (Fig. 2) und deren Anschlüssen 4 und 5 bzw. 7 und 8 in einer rohrförmigen Hülle 9 aus Glas oder dergleichen, beispielsweise auch Quarz.

Auf der Innenfläche 10 dieser Hülle 9 ist eine Reflexionsschicht 11 aufgebracht und anschließend von einer Leuchtstoffschicht 12 überdeckt worden, wobei vor dem Überdecken durch die Leuchtstoffschicht 12 dafür gesorgt wurde, daß zumindest ein Teil der Innenfläche 10 in Längsrichtung des Glasrohrs bzw. der Hülle 9 (siehe Längsachse 13) schlitzartig von der Reflexionsschicht 11 frei ist, derart, daß das im Betrieb aus dem vorhandenen Schlitz 14 (Fig. 1) austretende sichtbare und/oder UV-Licht durch die Reflexionsschicht 11 verstärkt ist.

Erfindungsgemäß endet nun der Schlitz 14 beidseitig, d.h. an beiden Enden der Hülle 9, vor den Elektroden 2 und 3, derart, daß ab jeweils Rohrende 15 bzw. 16 zumindest der Elektrodenbereich D bzw. D' der rohrförmigen Hülle 9 über 360° ihres Umfangs durch die Reflexionsschicht 11 abgedeckt ist (siehe Fig. 4).

Natürlich kann es zweckmäßig sein, diese Bereiche D bzw. D' sich etwas über die Längserstreckung der Elektroden 2 und 3 hinauserstrecken zu lassen, um umso zuverlässiger ein eventuelles Wendelflackern einerseits und auftretende Innenwandschwärzungen andererseits zu verdecken.

## Patentansprüche

1. Niederdruckentladungslampe (1) für Bräunungszwecke mit endseitigen Elektroden in einer rohrförmigen Hülle (9) auf deren Innenfläche (10) eine Reflexionsschicht (11) aufgebracht und anschließend von einer Leuchtstoffschicht (12) überdeckt worden ist, wobei vor dem Überdecken durch die Leuchtstoffschicht (12) dafür gesorgt wurde, daß zumindest ein Teil der Innenfläche (10) in Längsrichtung (13) des Rohrs schlitzartig von der Reflexionsschicht (11) frei ist, derart, daß das im Betrieb aus dem vorhandenen Schlitz (14) austretende sichtbare und/oder UV-Licht durch die Reflexionsschicht (11) verstärkt ist,
**dadurch gekennzeichnet, daß**
der Schlitz (14) beidseitig vor den Elektrodenenden (2, 3) endet, derart, daß ab jeweils Rohrende (15, 16) zumidest der Elektrodenbereich (D, D') der rohrförmigen Hülle (9) über 360 Grad ihres Umfangs durch die Reflexionsschicht (11) abgedeckt ist.

## Claims

1. Low pressure discharge lamp (1) for the purpose of tanning comprising electrodes at its ends in a tubular shaped envelope (9) on the interior surface (10) of which a reflective layer (11) has been deposited and subsequently overlaid by a layer of fluorescent material (12), whereby before effecting the overlaying with the fluorescent layer (12) it has been provided that at least a portion of the interior surface (10) in longitudinal direction (13) of the tube is free of the reflective layer (11) like a slot (14), so that the visible and/or UV light exiting from the existing slot is enhanced by the reflective layer (11),
**characterized in that**
the slot (14) ends on both sides before the ends (2, 3) of the electrodes, so that from each tube end (15,16) at least the electrode area (D, D') of the tube shaped envelope (9) is covered by the reflective layer (11) over 360 degrees of its circumference.

## Revendications

1. Lampe (1) à décharge à basse pression pour bronzer, comprenant des électrodes d'extrémité dans une enveloppe (9) tubulaire sur la surface (10) intérieure de laquelle a été déposée une couche (11) réfléchissante et qui a été ensuite revêtue d'une couche fluorescente (12) en faisant en sorte, avant le revêtement par la couche (12) fluorescente, qu'au moins une partie de la surface (10) intérieure dans la direction longitudinale (13) de tube soit, sous la forme d'une fente, dépourvue de la couche (11) réfléchissante, de manière à ce que la lumière visible et/ou UV sortant de la fente (14) soit renforcée par la couche (11) réfléchissante,
**caractérisée en ce que**
la fente (14) se termine des deux côtés avant les extrémités (2, 3) des électrodes, de manière à ce qu'à partir des extrémités (15, 16) du tube au moins la région d'électrodes (D, D') de l'enveloppe (9) tubulaire soit couverte sur 360 degrés de son pourtour par la couche (11) refléchissante.
